# EUROPEAN PATENT APPLICATION

(11) **EP 4 343 783 A1**
(43) Date of publication of application: **27.03.2024**
(21) Application number: 22197879.4
(22) Date of filing: 26.09.2022
(51) Int. Cl.: G16H 40/63, G06N 20/00

(54) **A SYSTEM FOR IMPLEMENTING AN AI-BASED SURGICAL SYSTEM**

(71) Applicant: Baxter Medical Systems GmbH + Co. KG, 07318 Saalfeld (DE)
(72) Inventor: DALBERT, Heinz-Hermann, 07318 Saalfeld (DE); LÖSER, Steffen, 07318 Saalfeld (DE); MAIER, Patrick, 07318 Saalfeld (DE); AMALANESAN, Antony L. Francis, 07318 Saalfeld (DE)
(74) Representative: Loustalan, Paul William

(57) **Abstract**

A system for implementing an AI-based surgical system configured to predict and execute steps of a surgical procedure, the system comprising: a prediction module configured to predict a step of a surgical procedure; an input configured to receive feedback on the prediction from a user and provide the feedback to the prediction module; an execution module configured to execute the step of the surgical procedure based on the prediction and based on the feedback when the system is operating under supervision; and a supervision module configured to validate and approve the system for unsupervised use based on one or more criteria.

## Description

### FIELD OF THE INVENTION

The present invention relates to a system for implementing an AI-based surgical system, and in particular, an AI-based surgical system configured to carry out steps of a surgical procedure. The present invention also relates to a method for implementing an AI-based surgical system, a supervision module for use in the surgical system, and a computer program.

### BACKGROUND OF THE INVENTION

With time, more and more AI-based algorithms and systems have the potential to find deployment inside an operating room (OR) or similar environment. Such systems are expected to use data occurring during surgery to autonomously make decisions, e.g. predict, advise, warn, and interact with human staff. Some examples include passively acting OR workflow prediction algorithms for workflow optimization purposes, or robotic systems who autonomously interact with the human staff based on Artificial Intelligence (AI)-based decisions.

Training such AI systems takes significant time, and when deploying such systems in applications such as operating rooms, their safety of operation must be ensured.

### SUMMARY OF THE INVENTION

The invention is defined by the independent claims to which reference should now be made. Optional features are set forth in the dependent claims.

The inventors of the present invention have appreciated the need for a sophisticated strategy to introduce AI-based systems into the OR and ultimately to enable such AI-based systems to act autonomously. Deployment of an AI-based system can be error-prone due to lack of robustness, a robust AI system being a system that has been verified mathematically and validated through testing, thereby ensuring its safety and functionality. False decisions of an AI algorithm will lead to errors within the OR such as an inappropriate instrument being provided to a surgeon. Such an error may not be noticed until a problem is caused resulting from the error. In addition, the error may be noticed by a human present in the OR but a user such as a surgeon is inconvenienced, time delays a caused, and intervention from human staff may be required.

According to embodiments described herein, a preliminary validation and approval period is provided for AI-based systems being introduced into the OR. In this period, at least one human staff, for example within the OR, supervises the AI-based system by being able to interpret and understand the decisions of the machine learning algorithm and having the ability to abort and correct the AI-based decisions. These corrections have the potential to be used for optimization purposes to increase robustness of the underlying AI algorithm. This approach allows for the introduction of new Al-based solutions into surgical workflows without requiring lengthy training outside of the OR and therefore when the Al-based system is not assisting with surgical procedures. The proposed approach also allows for optimization of the machine learning algorithms during their deployment, and validation and approval of the systems for autonomous deployment inside the OR. Once a system is approved, the system may act autonomously in the OR, and may also supervise or train human staff.

According to an aspect of the present invention, there is provided a system for implementing an AI-based surgical system configured to predict and execute steps of a surgical procedure, the system comprising: a prediction module configured to predict a step of a surgical procedure; a feedback module configured to receive feedback on the prediction from a user input and provide the feedback to the prediction module; an execution module configured to execute the step of the surgical procedure based on the prediction and based on the feedback when the system is operating under supervision; and a supervision module configured to validate and approve the system for unsupervised use based on one or more criteria.

In some examples, the prediction module evaluates incoming data when predicting a step of the surgical procedure. The incoming data may non-exhaustively include one or more of: hospital records, patient information, historical surgical information (which may be based on surgeries carried out with or without the assistance of the AI-based surgical system), and data manually input by users. In some examples, the prediction module is configured to predict the step of the surgical procedure based at least in part on historical surgical data. The prediction module may provide the prediction with a corresponding probability. In some examples, if the probability exceeds a predetermined threshold, a request may be sent to the execution module to perform the predicted step (for example, a request is sent and the execution module instructs a user such as an OR nurse to prepare the respective instrument). In some examples, the execution module is configured to control a surgical robot to execute the step of the surgical procedure. Before the request is carried out, the request is subject to feedback from a user through the feedback module when operating in unsupervised mode. When the AI-based surgical system is validated and approved for unsupervised operation, the request may be sent directly to the execution module via the supervision module to be carried out without feedback from a user.

In some examples, the prediction module is configured to use a machine learning model of the surgical system to predict the surgical step. In some examples, the machine learning model of the surgical system is configured to be trained at least in part by the feedback.

Through the feedback module, human staff are given the ability to understand the ideas and decisions of the predictor, as well as being provided the ability to intervene in required cases. That is, the feedback module comprises a user interface configured to display information to one or more users. This may be, for example, a display for displaying predictions made by the predictor. the feedback module may comprise a computer device such as a desktop computer, laptop computer, or tablet computer providing user input. The user interface also comprises a user input configured to allow the user to provide feedback on a prediction made by the predictor. In this example, the user input is a touch screen display. The touch screen display displays feedback options including acceptance of a prediction, non-acceptance of a prediction, and the ability to alter the prediction or suggest a new surgical step to be performed. In addition, the user interface enables a user to initiate a surgical step. For example, where a surgical step is required by the predictor has not made a prediction, the human staff may manually input that a step is required, which is then executed by the execution module. Based at least in part on the feedback and any user inputs provided to the feedback module, the machine learning algorithm may be trained.

The feedback module may comprise any appropriate user interface to enable input of feedback from a user. In some examples, the feedback comprises one or more of: an indication to continue with the predicted step; an indication not to continue with the predicted step; and an alteration to the predicted step to provide an alternative surgical step. The predicted next step of a surgical procedure may comprise not performing an action, or in some examples, the prediction module may not perform a prediction. This may be determined to be incorrect and a next surgical step may be input or initiated by through the user input or user interface to the feedback module and sent to the execution module to execute the initiated step of the procedure. The prediction module may predict a plurality of steps of the surgical procedure, and each request may be displayed with a corresponding probability. Alternatively, the most probable predicted step may be displayed with a corresponding probability, and alternative steps may also be displayed.

A user may then evaluate the predicted step or steps and, if required, correct the prediction module's prediction by selecting another alternative. In some embodiments, it may be advantageous to have a designated user such as a human nurse tasked specifically to supervise the system. The designated user will have high experience in the role of the surgical robot (scrub nurse in this example) and will be: the main instructor of the system during the introduction and training of the AI in the OR; managing incoming interventions by other users such as sterile staff; and the first contact for the sterile staff regarding issues with the AI system.

The feedback provided by the user may be sent to the supervision module which may record the feedback received. The supervision module acts as an intermediary between the prediction module running an AI algorithm and the execution module. Where a request is corrected or overwritten by a user via the feedback module, the corrected request is sent to the execution module. Alternatively, the user may accept or deny the request, and the request is then sent or not sent to the execution module respectively.

In some examples, the one or more criteria comprises a number of corrected errors against predictions made by the prediction module. That is, the supervision module may record the number of corrected predicted steps made by the prediction module and record the number of predicted steps against the number of predictions made. In some examples, once a predetermined ratio of corrected steps against predicted steps is reached, the AI-based surgical system may be validated and approved for unsupervised use. To reach this predetermined ratio, the prediction module may be required to have performed a predetermined number of predictions to ensure robustness of the system.

In some examples, the one or more criteria also or alternatively comprises the time taken for the prediction module to make the prediction. In applications such as an OR, time is valuable and predictions must be made quickly. A module, which may be the prediction module or separate module, may record the time taken for the prediction module to make a prediction. The supervision module may be configured only to validate or approve the prediction module for unsupervised use when the prediction module makes predictions below a predetermined threshold time. Statistical data may be used to compare the time taken for user predictions or decisions with the prediction modules predictions in order to determine the predetermined threshold.

In order to evaluate based on additional criteria, additional data may be required. This data may be input manually by a user, and the one or more criteria may be based at least in part on the manually input data.

In some examples, the supervision module may validate and approve the AI-based surgical system for unsupervised operation only in particular operating conditions. For example, the supervision module may validate and approve the system for unsupervised use under normal operating conditions, but the system is not validated and approved for unsupervised use under extreme conditions such as an emergency situation. That is, differing levels of validation and approval may be provided, each requiring one or more criteria to be met in order to be approved for unsupervised use. For example, a system may need to operate under supervision in an emergency situation a predetermined number of times before being approved for unsupervised use in an emergency situation.

In some examples, having been validated and approved for unsupervised operation, the Al-based surgical system may be used to train or supervise human users. This provides a mutual supervision framework for the Al-based surgical system interacting with users such as human OR staff.

According to another aspect of the invention, there is provided a method for implementing an AI-based surgical system configured to predict and execute steps of a surgical procedure, the method comprising: an AI-based module predicting a step of a surgical procedure; providing feedback on the prediction to the AI-based module; executing the step of the surgical procedure based on the prediction and based on the feedback when the system is operating under supervision; and validating and approving the system for unsupervised use based on one or more criteria.

In some examples, the one or more criteria comprises a number of corrected errors against predictions made by the prediction module.

In some examples, the feedback comprises one or more of: an indication to continue with the predicted step; an indication not to continue with the predicted step; and an alteration to the predicted step to provide an alternative surgical step.

In some examples, the method further comprises using a machine learning model of the surgical system to predict the surgical step. In some examples, the method further comprises training the machine learning model of the surgical system at least in part based on the feedback.

In some examples, the executing the step of the surgical procedure comprises controlling a surgical robot to execute the step of the surgical procedure.

In some examples, the predicting the step of the surgical procedure comprises the Al-based module predicting the step based at least in part on historical surgical data.

According to another aspect of the present invention, there is provided a computer program for performing the method according to embodiments described herein.

According to another aspect of the present invention, there is provided a non-transitory computer-readable medium having stored thereon instructions that, when executed by a processor, cause the processor to perform the steps of the method according to embodiments described herein.

According to another aspect of the present invention, there is provided a supervision module for use in the system according to embodiments described herein.

According to another aspect of the present invention, there is provided a supervision module for implementing an AI-based surgical system configured to predict and execute steps of a surgical procedure, the supervision module comprising: one or more inputs configured to receive an AI-based prediction of a step of a surgical procedure and to receive feedback on the prediction from a user; and an output configured to output an instruction for executing the step of the surgical procedure based on the prediction and based on the feedback when the system is operating under supervision, the supervision module being configured to validate and approve the AI-based surgical system for unsupervised use based on one or more criteria. The one or more inputs may be any suitable communication connection suitable for connecting, for example, to a prediction module and execution module as described herein. The feedback received from a user may be received through a feedback module, which may be part of the supervision module or may be a separate module in communication connection with the supervision module.

According to another aspect of the present invention, there is provided method for implementing an AI-based surgical system configured to predict and execute steps of a surgical procedure, the method comprising: providing an AI-based prediction of a step of a surgical procedure; providing feedback on the prediction; providing an instruction for executing the step of the surgical procedure based on the prediction and based on the feedback when the system is operating under supervision; and validating and approving the system for unsupervised use based on one or more criteria.

### BRIEF DESCRIPTION OF THE DRAWINGS

The invention will now be described in more detail, by way of example, with reference to the accompanying drawings, in which:
Figure 1 is a block diagram of an AI-based surgical system embodying an aspect of the present invention;
Figure 2 is a schematic diagram of an operating room for applying an AI-based surgical system embodying an aspect of the present invention;
Figure 3 is a schematic diagram of an AI-based surgical system implemented in an operating room embodying an aspect of the present invention;
Figure 4 is a block diagram of an AI-based surgical system embodying an aspect of the present invention in use;
Figure 5 is a block diagram of an AI-based surgical system embodying an aspect of the present invention in use;
Figure 6 is a block diagram of an AI-based surgical system embodying an aspect of the present invention in use;
Figure 7 is a block diagram of an AI-based surgical system embodying an aspect of the present invention in use; and
Figure 8 is a schematic diagram of an AI-based surgical system implemented in an operating room embodying an aspect of the present invention.

Like features are denoted by like reference numerals.

### DETAILED DESCRIPTION

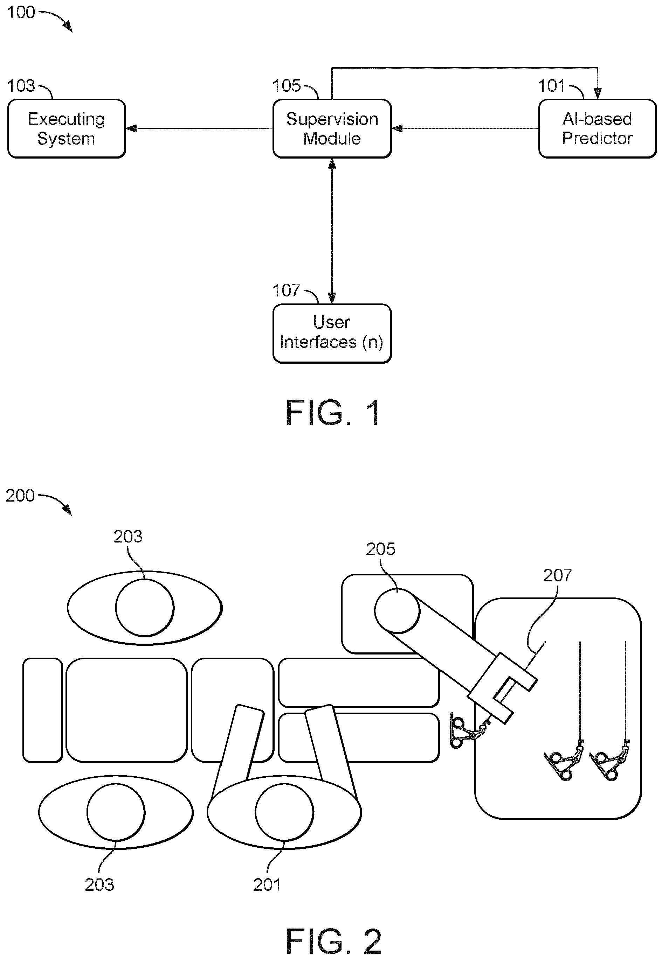

An example system and method for implementing an Al-based surgical system in an operating room for unsupervised use will now be described with reference to Figures 1 to 7.

Figure 1 illustrates an AI-based surgical system 100 embodying an aspect of the present invention. The system 100 comprises a prediction module which here is an AI-based predictor 101. The predictor 101 is in communication connection with an execution module or executing system 103 via a supervision module 105. The supervision module 105 is in communication connection with a feedback module which here comprises user interfaces 107.

The supervision module receives information provided by the prediction module or AI-based predictor such as a current prediction or decision, or interpretable variables. The supervision module processes the data and sends human-interpretable information to the user interfaces to be displayed.

Feedback is provided by the user through the user interfaces of the feedback module. The feedback may include interventions such as verifications, initiations, and corrections. In particular, the feedback may comprise one or more of: an indication to continue with the predicted step; an indication not to continue with the predicted step; and an alteration to the predicted step to provide an alternative surgical step.

The supervision module accepts the feedback from the user and sends verified decisions to the execution module or executing system. In addition, the supervision module sends data related to the feedback to the AI-based predictor such that the machine learning algorithm used by the AI-based predictor can be trained at least in part based on the feedback.

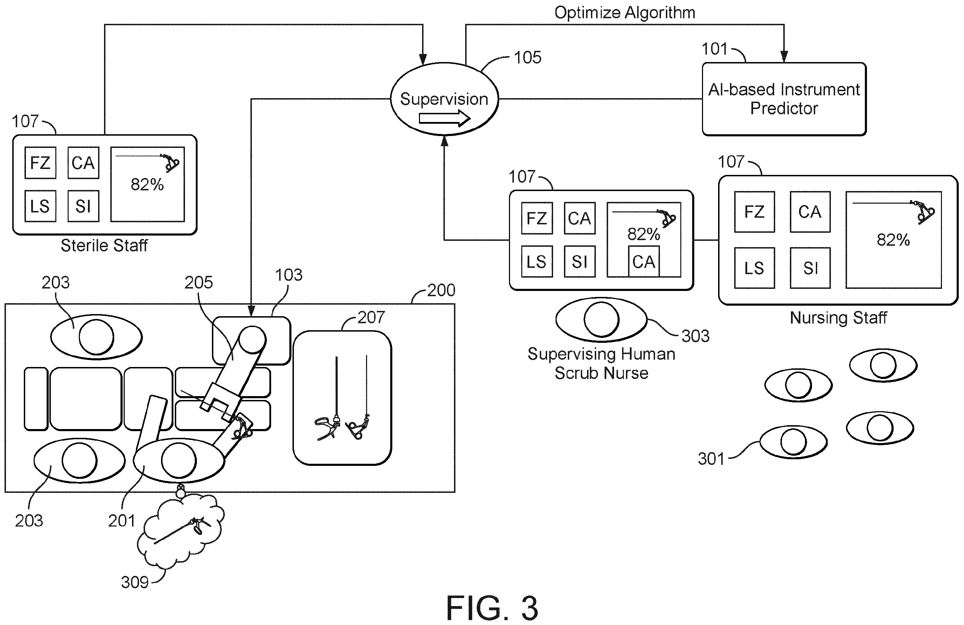

Figure 2 illustrates an operating room 200 in which the system 100 may be implemented. The operating room 200 comprises a surgeon 201 and nurses 203. The operating room 200 also comprises a robotic scrub nurse 205 configured to assist with surgery by selecting and handing instruments 207 to the surgeon 201. As will be described in more detail with respect to Figure 3, the scrub nurse 205 may be controlled by the AI-based surgical system according to embodiments described herein.

Figures 3 illustrates the operating room 200 of Figure 2 with the AI-based surgical system 100 implemented to, in this example, control the scrub nurse 205.

Broadly, the method for implementing the AI-based surgical system into an operating room may be viewed as comprising three phases as follows. The first phase comprises introducing an AI-based system with limited robustness into the OR. In this initial stage, the AI-based surgical system operates under supervision or in a supervision mode. In this mode, the system may predict a surgical step which is viewed by a user and feedback provided on the prediction. This feedback may be acceptance, denial, correction of a particular surgical step predicted, or initiation of a surgical step, the feedback then being used to further train the AI algorithm.

In a second phase, the system is validated and approved for unsupervised use having been analysed according to one or more criteria. For example, when a number of corrected errors made against predicted steps reaches a particular ratio or percentage, the system may be determined to be ready for unsupervised use and is thus validated and approved for such use. It will be appreciated that this criteria is one example criteria and any suitable criteria may be used which would sufficiently confirm the safety and robustness of the AI and of operation of the AI-based surgical system.

Lastly, in a third phase, the AI-based system operating in unsupervised mode is used for assistance with surgical procedures without requiring feedback for operation. In addition, the AI-based system operating in unsupervised mode may also be used for training staff, helping to decrease their proneness to error.

In more detail, the AI-based surgical system is a system 100 which autonomously interacts based on inputs by AI algorithms. In this example, the AI-based surgical system comprises a robotic scrub nurse 205 which acts in the sterile OR field and is able to detect and flexibility manipulate different instruments 207 in the OR. The robotic scrub nurse 205 comprises an interface by which the instrument to be detected and manipulated can be communicated to a user.

It will be appreciated that, while in this example the AI-based surgical system comprises a robotic scrub nurse, in other implementations, the AI-based surgical system may comprise any system for autonomously interacting based on inputs by AI algorithms.

The predictor 101 predicts a next step of a surgical procedure. In this example, the next step comprises a suggestion for an instrument to be used. The instrument will be passed to the surgeon by the scrub nurse.

However, in order to implement the system 100 ultimately for unsupervised use, the system 100 first undergoes a period of operation under supervision. There is a chance that the Al-based predictor will predict an incorrect step of the surgical procedure. In this example, this would be an incorrect identification of a required instrument. The surgeon would then be passed the incorrect instrument and may not notice before using it, potentially causing a problem during the procedure. This may occur when the surgeon assumes to have received a particularly instrument 309 but instead receives a different instrument. For example, the predictor may suggest use of scissors instead of pliers, or vice versa. Even if the surgeon notices the incorrect instrument, inconvenience and time delay is caused.

Supervision is therefore required, providing an intermediary between the AI-based predictor and an execution module controlling the scrub nurse to perform steps of the surgical procedure.

Significantly, the system 100 comprises the supervision module 105 acting as the intermediary between the predictor 101 and the execution module 103. In this way, the prediction made by the predictor 101 can be evaluated by 101 in order to ensure the safety of a procedure in the operating room 200.

The predictor 101 forms a prediction for a next surgical step based on evaluation of data. In this example, the predictor 101 evaluates historical surgical data to form the prediction, but it will be appreciated that any suitable data may be used including hospital records, patient information, and data based on the current procedure being performed. The predictor 101 uses a machine learning algorithm of the system to form the prediction.

The predictor 101 forms the prediction and the prediction is displayed on user interfaces 107 of the feedback module in a human-readable manner. In particular, in this example, the prediction is displayed with a corresponding probability, together with alternative potential surgical steps. In order for the predicted surgical step to be sent from the predictor 101, the corresponding probability must exceed a predetermined threshold, which may be set by a user prior to the surgical procedure.

The prediction is viewed and evaluated by one or more users. In this example, nursing staff 301 review the prediction and provide feedback through a selection on the user interface 107. A supervising human scrub nurse 303 reviews the prediction and the feedback provided by the nursing staff 301 and provides feedback, which may be in agreement with the nursing staff 301 of may override the nursing staff's feedback. The feedback provided by the human scrub nurse 303 is provided to the supervision module 105 together with the initial prediction.

In addition, in this example, sterile staff 203 within the operating room also provide feedback through a user interface 307 which is provided to the supervision module.

The feedback provided by users is passed from the supervision module 105 back to the predictor 101 and is used to train and optimize the machine learning algorithm of the predictor 101 used to form predictions.

The execution module 103 then executes a step of a surgical procedure based on the prediction and, when operating under supervision, based on the feedback received. Where the feedback confirms the predicted step, the execution module 103 controls the robotic scrub nurse 205 to carry out the predicted step. Where the feedback denies the predicted step, the execution module 103 does not carry out the predicted step. Where the feedback indicates a corrected or alternative step, the execution module 103 controls the robotic scrub nurse 205 to carry out the corrected or alternative step. Lastly, where the prediction module does not form a prediction but it is determined by a user that a step is necessary, the step is initiated via the feedback module to the supervision module 105 and execution module 103 to control the robotic scrub nurse to carry out the initiated surgical step.

The system 100 operates under supervision until the supervision module 105 determines that the system 100 should be validated and approved for unsupervised use based on one or more criteria. In this example, the one or more criteria comprises a number of corrected predictions against predictions made. Initially, a predetermined threshold of number of predictions must be met. This threshold may be set by a user. Once this threshold has been met, once a ratio of corrected predictions against predictions made reaches a predetermined ratio, the system 100 is validated and approved for unsupervised use. The system 100 may then assist or provide guidance during surgical procedures without requiring feedback from the feedback module. That is, a prediction is made by the predictor 101 and may be passed to the execution module 103 to control the robotic scrub nurse 205 without requiring user input via the feedback module.

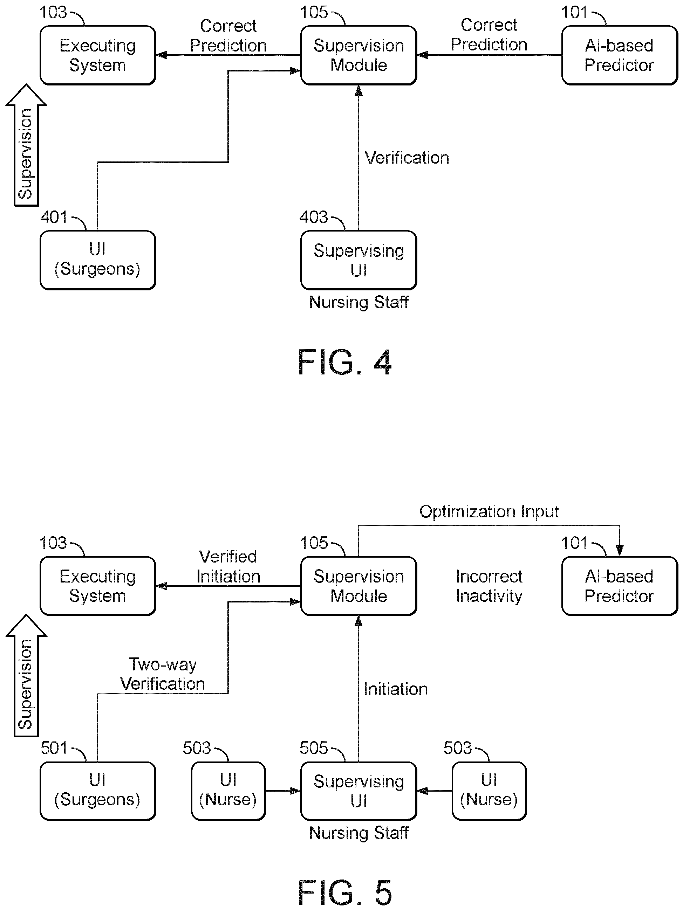

Figures 4 to 6 illustrate block diagrams of different feedback scenarios. Figure 4 illustrates a scenario in which the predictor 101 predicts a correct surgical step. The predicted step is passed to the supervision module 105 and reviewed via the feedback module by users including, in this example, surgeons and nursing staff. Each of the surgeons and nursing staff provide feedback via user interfaces 401 and 403 respectively, confirming the prediction. The correct prediction is provided to the execution module 103, which subsequently controls the robotic scrub nurse to carry out the predicted step.

This is an example of active verification requiring user inputs via the user interfaces in order to verify the correct surgical step. It will be appreciated that passive verification may also be implemented in which the users may review the predicted step but not necessarily provide feedback in the form of an input.

Figure 5 illustrates a scenario in which the predictor 101 has failed to provide a prediction. This may be due to a predicted step being missed, or because a prediction failed to meet the predetermined probability threshold. In this scenario, the nursing staff indicate that a surgical step is required by providing feedback to the feedback module through user interfaces. In this example, members of nursing staff 503 provide feedback which is initially reviewed by a supervising nurse 505, who then provides feedback to the supervision module. Other users such as surgeons 501 may review and verify the surgical step proposed by the nursing staff. It will be appreciated that any user may indicate that a surgical step has been missed and initiate a necessary step.

Once verified, the supervision module 105 provides the surgical step to be executed to the execution module 103 which executes the surgical step as indicated. The supervision module 105 provides the feedback to the predictor 101 to train the algorithm used for basing predictions, reducing the possibility of another prediction being missed by the predictor 101.

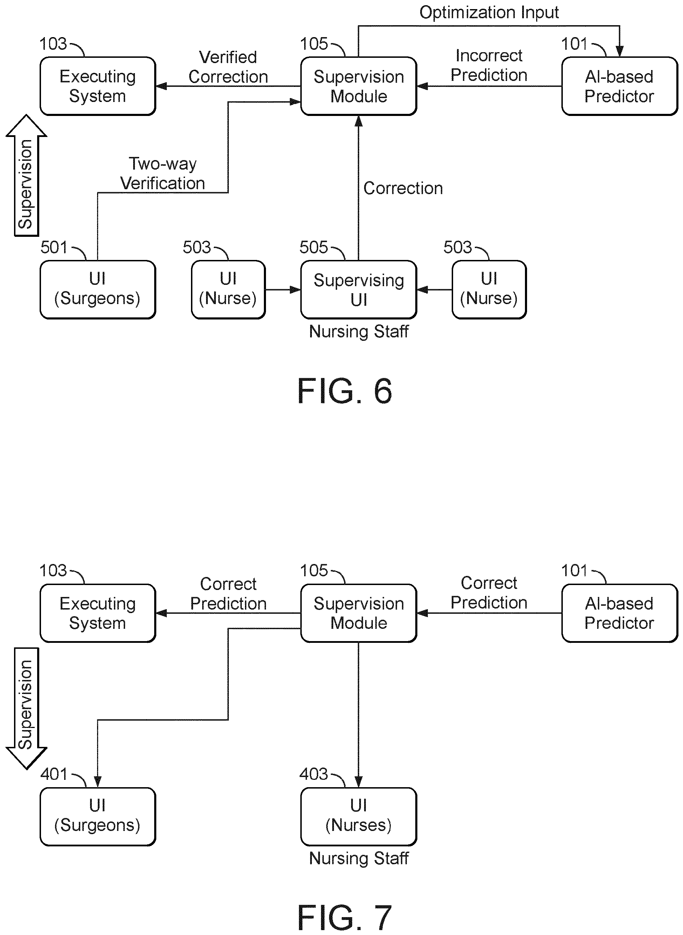

Figure 6 illustrates a scenario in which the predictor 101 predicts an incorrect surgical step. The predicted step is provided to the supervision module 105 and is reviewed via the feedback module by users including nursing staff 503 and a supervising nurse 505. The users determine that the predicted step is incorrect and provide feedback correcting the suggested step. The feedback provided may be reviewed and verified by a user such as a surgeon 501. The corrected or alternative step is passed to the supervision module 105 and subsequently to the execution module 103 for execution. Again, the supervision module 105 sends the feedback to the predictor 101 for training and optimizing the algorithm. It will be appreciated that the error and feedback may be provided by another user such as the surgeon 501.

Figure 7 illustrates implementation of the validated and approved AI-based surgical system operating in unsupervised mode and which may be used for guidance of medical staff and a learning possibility for inexperienced staff, as well as an assistance system to prevent human error. A correct prediction is made by the predictor 101 which is passed to the supervision module 105. The supervision module 105 displays the correct prediction to users including nursing staff and surgeons who can be trained on correct procedure based on the predictions made. This provides guidance to staff as well as preventing incorrect decisions during a procedure. The correct predicted step is then passed to the execution module 103 for execution. Thus, a mutual supervision framework is provided in which supervision is initially provided to the system in the initial training phase, and then provided to users by the Al-based surgical system once validated and approved. This is made possible as the predictor AI has been validated as being robust enough for safe and secure operation with the operating room through the methods of implementation described herein. Advantageously, the validation and approval is performed during practical operation within the operating room, removing the need for a lengthy training process outside of a practical application.

It will be appreciated that the unsupervised system does not need to be used to train human users. It will also be appreciated that, once validated and approved, the prediction need not be displayed, but the predicted step can be passed through the supervision module 105 to the execution module 103 for execution.

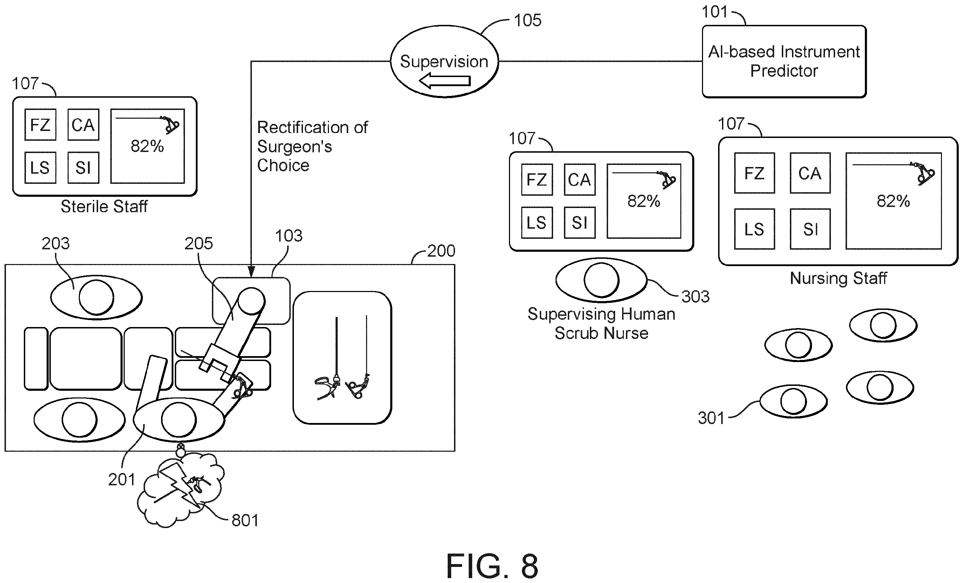

A more detailed illustration of using the unsupervised AI for guiding medical staff in an operating room 200 is provided in Figure 8.

The predictor 101 makes a correct prediction of the next surgical step to be carried out which is passed to the supervision module 105, now operating to supervise human users rather than the predictor 101. The correct prediction is displayed on user interfaces 107 to nursing staff 301, a supervising scrub nurse 303, and to sterile staff 203 within the OR. Initially, a surgeon 201 may have pre-selected a surgical step such as a particular instrument 801 before receiving the prediction from the predictor 101. Having received the prediction being displayed via user interfaces 107, it is noticed that correctly predicted step from the predictor 101 is different to the step chosen by the surgeon, indicating a potentially incorrect step chosen by the surgeon. The surgeon and staff may then rectify the choice made based on the predicted step displayed, and the robotic scrub nurse 205 is controlled by the execution module 103 to pass the surgeon 201 the correct instrument, thereby avoiding a potential problem with the procedure due to use of an incorrect instrument. Alternatively, the users or staff could await instruction from the predictor 101 before beginning to carry out surgical steps.

Embodiments of the present invention have been described. It will be appreciated that variations and modifications may be made to the described embodiments within the scope of the present invention.

Although the above example has been described with reference to a system for use in an OR, it will be clear from the foregoing explanation embodiments of the invention may advantageously be used in any environment where an AI-based system is to be implemented and in which robustness of the AI is necessary (such as for safety concerns).

From the foregoing, it will be appreciated that the system may comprise a computer processor running one or more server processes for communicating with client devices. The server processes comprise computer readable program instructions for carrying out the operations of the present invention. The computer readable program instructions may be or source code or object code written in or in any combination of suitable programming languages including procedural programming languages such as C, object orientated programming languages such as C#, C++, Java, scripting languages, assembly languages, machine code instructions, instruction-set-architecture (ISA) instructions, and state-setting data.

The wired or wireless communication networks described above may be public, private, wired or wireless network. The communications network may include one or more of a local area network (LAN), a wide area network (WAN), the Internet, a mobile telephony communication system, or a satellite communication system. The communications network may comprise any suitable infrastructure, including copper cables, optical cables or fibres, routers, firewalls, switches, gateway computers and edge servers.

The system described above may comprise a Graphical User Interface. Embodiments of the invention may include an on-screen graphical user interface. The user interface may be provided, for example, in the form of a widget embedded in a web site, as an application for a device, or on a dedicated landing web page. Computer readable program instructions for implementing the graphical user interface may be downloaded to the client device from a computer readable storage medium via a network, for example, the Internet, a local area network (LAN), a wide area network (WAN) and/or a wireless network. The instructions may be stored in a computer readable storage medium within the client device.

As will be appreciated by one of skill in the art, the invention described herein may be embodied in whole or in part as a method, a system, or a computer program product including computer readable instructions. Accordingly, the invention may take the form of an entirely hardware embodiment or an embodiment combining software, hardware and any other suitable approach or apparatus.

The computer readable program instructions may be stored on a non-transitory, tangible computer readable medium. The computer readable storage medium may include one or more of an electronic storage device, a magnetic storage device, an optical storage device, an electromagnetic storage device, a semiconductor storage device, a portable computer disk, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk.

Exemplary embodiments of the invention may be implemented as a circuit board which may include a CPU, a bus, RAM, flash memory, one or more ports for operation of connected I/O apparatus such as printers, display, keypads, sensors and cameras, ROM, a communications sub-system such as a modem, and communications media.

In addition, the above detailed description of embodiments of the invention are not intended to be exhaustive or to limit the invention to the precise form disclosed. For example, while processes or blocks are presented in a given order, alternative embodiments may perform routines having steps, or employ systems having blocks, in a different order, and some processes or blocks may be deleted, moved, added, subdivided, combined, and/or modified. Each of these processes or blocks may be implemented in a variety of different ways. Also, while processes or blocks are at times shown as being performed in series, these processes or blocks may instead be performed in parallel, or may be performed at different times.

The teachings of the invention provided herein can be applied to other systems, not necessarily the system described above. The elements and acts of the various embodiments described above can be combined to provide further embodiments.

While some embodiments of the inventions have been described, these embodiments have been presented by way of example only, and are not intended to limit the scope of the disclosure. Indeed, the novel methods and systems described herein may be embodied in a variety of other forms; furthermore, various omissions, substitutions and changes in the form of the methods and systems described herein may be made without departing from the spirit of the disclosure.

## Claims

1. A system for implementing an AI-based surgical system configured to predict and execute steps of a surgical procedure, the system comprising:
a prediction module configured to predict a step of a surgical procedure;
an input configured to receive feedback on the prediction from a user and provide the feedback to the prediction module;
an execution module configured to execute the step of the surgical procedure based on the prediction and based on the feedback when the system is operating under supervision; and
a supervision module configured to validate and approve the system for unsupervised use based on one or more criteria.

2. The system of claim 1 wherein the one or more criteria comprises a number of corrected errors against predictions made by the prediction module.

3. The system of claim 1 or claim 2 wherein the feedback comprises one or more of: an indication to continue with the predicted step; an indication not to continue with the predicted step; and an alteration to the predicted step to provide an alternative surgical step.

4. The system of any of claims 1 to 3 wherein the prediction module is configured to use a machine learning model of the surgical system to predict the surgical step.

5. The system of claim 4 wherein the machine learning model of the surgical system is configured to be trained at least in part by the feedback.

6. The system of any of claims 1 to 5 wherein the execution module is configured to control a surgical robot to execute the step of the surgical procedure.

7. The system of any of claims 1 to 6 wherein the prediction module is configured to predict the step of the surgical procedure based at least in part on historical surgical data.

8. A supervision module for use in the system of any of claims 1 to 7.

9. A method for implementing an AI-based surgical system configured to predict and execute steps of a surgical procedure, the method comprising:
an AI-based module predicting a step of a surgical procedure;
providing feedback on the prediction to the AI-based module;
executing the step of the surgical procedure based on the prediction and based on the feedback when the system is operating under supervision; and
validating and approving the system for unsupervised use based on one or more criteria.

10. The method of claim 9 wherein the one or more criteria comprises a number of corrected errors against predictions made by the prediction module.

11. The method of claim 9 or claim 10 wherein the feedback comprises one or more of: an indication to continue with the predicted step; an indication not to continue with the predicted step; and an alteration to the predicted step to provide an alternative surgical step.

12. The method of any of claims 9 to 11 further comprising using a machine learning model of the surgical system to predict the surgical step.

13. The method of claim 12 further comprising training the machine learning model of the surgical system at least in part based on the feedback.

14. The method of any of claims 9 to 13 wherein the executing the step of the surgical procedure comprises controlling a surgical robot to execute the step of the surgical procedure.

15. A computer program for performing the method of any of claims 8 to 14.
